(19) Europäisches Patentamt
European Patent Office
Office européen des brevets

(11) **EP 3 151 023 A1**

(12) **EUROPEAN PATENT APPLICATION**
published in accordance with Art. 153(4) EPC

(43) Date of publication:
**05.04.2017 Bulletin 2017/14**

(51) Int Cl.:
***G01R 31/01*** *(2006.01)*

(21) Application number: **15799929.3**

(86) International application number:
**PCT/KR2015/005297**

(22) Date of filing: **27.05.2015**

(87) International publication number:
**WO 2015/182983 (03.12.2015 Gazette 2015/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **30.05.2014 KR 20140066214**

(71) Applicant: **i-Sens, Inc.**
**Seoul 137-873 (KR)**

(72) Inventors:
• **KIM, Tae Eun**
**Namyangju-si, Gyeonggi-do 472-877 (KR)**
• **KIM, Jin Ho**
**Goyang-si, Gyeonggi-do 412-824 (KR)**
• **PARK, Hyo Seon**
**Seocho-gu, Seoul 137-952 (KR)**
• **CHA, Geun Sig**
**Seoul 120-841 (KR)**
• **NAM, Hak hyun**
**Gangbuk-gu, Seoul 142-742 (KR)**

(74) Representative: **Groth & Co. KB**
**P.O. Box 6107**
**102 32 Stockholm (SE)**

(54) **MEASUREMENT ERROR CORRECTION DEVICE OF BIO-MEASURER**

(57) The present invention relates to a measurement error correction device of a bio-measurer and, more specifically, provides a measurement correction device which: enables a device for measuring a measurement error to be arranged inside a bio-measurer such that a measurement error can be corrected after the bio-measurer is manufactured; and enables a user to directly confirm, automatically or by the user's selection, whether the bio-measurer is normally operated at an early stage of the operation of the bio-measurer, thereby reducing the time and the cost for correcting a measurement error during a manufacturing process of the bio-measurer.

FIG.2

## Description

## TECHNICAL FIELD

The present disclosure relates to a measurement error correction device of a bio-measurer. More particularly, the present disclosure relates to a measurement error correction device able to correct a measurement error of a bio-measurer after the bio-measurer is fabricated using a measurement error-detecting device disposed within the bio-measurer and allowing a user to recognize whether or not the bio-measurer ordinarily operates in an early stage, automatically or by a user selection, thereby reducing time and cost spent for correcting the measurement error in the fabrication of the bio-measurer.

## BACKGROUND ART

Diabetes is one of worldwide main causes of death and physical disability, and a significantly large number of people are suffering from diabetes. Complications from diabetes include heart and kidney diseases, loss of eyesight, nerve damage, and hypertension, and a significant amount of costs are consumed thereby. Long-term clinical studies have found that the start of complications can be significantly reduced when the level of blood glucose is suitably regulated. Thus, an important requirement in the management of diabetes is that a diabetic monitor his or her level of blood glucose at any time and in any place.

Due to such demand, biometric value self-measuring systems with which users can inspect levels of blood glucose have been widely distributed and used. In general, a bio-measurer, such as a blood glucose meter, is a device inspecting health conditions of a user by measuring the biometric values of the user, such as a level of blood glucose. That is, the biometric values of the user are measured by applying an object to be measured on a sensor strip (i.e. a bio-sensor strip). For example, in the case of the blood glucose meter, a sensor strip of the blood glucose meter is fitted into a slit, and a small amount of blood is gathered from the tip of a finger. When the gathered blood is brought into contact with the sensor strip of the blood glucose meter, the blood is automatically absorbed into the slit of the sensor strip, and then a measured value of blood glucose is output on a display.

The blood glucose meter measures a value of blood glucose from an electric signal generated by an electrochemical reaction between the gathered blood and a reactant in the sensor strip. However, a measurement error occurs due to a fabrication process of the blood glucose meter or a fabrication process of the sensor strip. A process of correcting a measurement error of the blood glucose meter or a measurement error of the sensor strip is required, since the blood glucose meter typically measures the blood glucose of a user using a minute electrochemical reaction. The range of the measurement error of the sensor strip of each sensor is determined in the fabrication process of the sensor strip, in which the accuracy of the process of measuring the level of blood glucose is determined. Information regarding correction due to an error of each sensor strip is input in the fabrication of the sensor strip, so that the error of the sensor strip is corrected using information regarding correction obtained from the sensor strip.

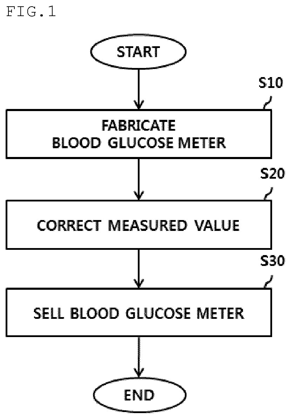

A related-art process of correcting a measurement error of a blood glucose meter will be described with reference to FIG. 1. When the fabrication of a blood glucose meter is completed (S10), a measurement error of the blood glucose meter is corrected by comparing an actual level of blood glucose obtained by measuring blood glucose from a standard sample using the fabricated blood glucose meter with the level of blood glucose of the standard sample (S20). That is, the standard sample is a sample having a determined level of blood glucose. The actual level of blood glucose of the standard sample is measured by blotting the standard sample on the sensor strip, and a measurement error of the blood glucose meter is corrected based on the measured level of blood glucose and the determined level of blood glucose of the standard sample, so that the measurement error of the blood glucose meter is removed.

When the measurement error of the blood glucose meter is corrected, a standard sample is finally re-measured. An actual measurement level of blood glucose is determined whether or not to be in a critical range of blood glucose of the standard sample. When the re-measured level of blood glucose of the sample is in the critical range of blood glucose, the blood glucose meter is determined to be ordinary and then is sold (S30).

## DISCLOSURE

## Technical Problem

According to the method of correcting the measurement error of the blood glucose meter as described above, the measurement error of the blood glucose meter must be corrected using a standard sample during the fabrication process, and after the correction of the measurement error is completed, the blood glucose meter must be retested whether or not the blood glucose meter ordinarily measure the level of blood glucose. Thus, the process of correcting the measurement error of each bio-measurer using the separate standard sample requires a large amount of time and labor, thereby contributing to increased fabrication costs of blood glucose meters.

When a user uses a blood glucose meter that he or she purchased, it is difficult for the user to determine whether or not the blood glucose meter ordinarily operates. When the level of blood glucose temporarily increases or decreases, the user re-measures the level of blood glucose by recollecting blood, which is problematic. In addition, in the blood glucose meter of the related art, the user cannot trust the level of blood glucose measured using the blood glucose meter that he or she possesses, since it is impossible to inspect whether or not the blood glucose meter ordinarily operates. Thus, the user typically inquires a manufacturer or requests the manufacturer for an after service (A/S). In this case, the manufacturer may spend a large amount of labor and cost to respond to the user inquiry or perform A/S for the blood glucose meter that ordinarily operates.

Accordingly, the present disclosure has been made in consideration of the above problems occurring in the related art, and the present disclosure proposes a measurement error correction device of a bio-measurer, the measurement error correction device having a measurement error-detecting device disposed within the bio-measurer to automatically correct a measurement error of the bio-measurer when electric power is initially applied to the bio-measurer after the bio-measurer is fabricated.

Also proposed is a measurement error correction device of a bio-measurer, the measurement error correction device having a measurement error-detecting device disposed within the bio-measurer to reduce time and cost spent for correcting the measurement error in the fabrication of the bio-measurer.

Also proposed is a measurement error correction device of a bio-measurer, the measurement error correction device allowing a user to recognize whether or not the bio-measurer ordinarily operates in an early stage, automatically or by a user selection.

**Technical Solution**

According to an aspect of the present disclosure, a measurement error correction device of a bio-measurer may include: a first simulator having a first resistance value; a second simulator having a second resistance value; a measurement section measuring a first current value by applying electric power to the first simulator and measuring a second current value by applying electric power to the second simulator; and a correction section correcting a measurement error of the bio-measurer using the first current value and the second current value.

In the measurement error correction device, the correction section may correct the measurement error of the bio-measurer by measuring the first current value and the second current value in an error correction mode in which an error of the bio-measurer is corrected. The correction section may calculate a correction slope and a correction size from the measured first current value, the measured second current value, a first theoretical current value corresponding to the first resistance value and applied electric power, and a second theoretical current value corresponding to the second resistance value and applied electric power.

It is preferable that the measurement error correction device further includes: a third simulator having a third resistance value; and an operation inspection section measuring a third current value by applying electric power to the third simulator and inspecting whether or not the bio-measurer ordinarily operates based on the third current value in a user mode in which it is inspected whether or not the bio-measurer ordinarily operate.

It is preferable that the first simulator, the second simulator, the measurement section, the correction section, and the third simulator of the measurement error correction device are disposed within the bio-measurer.

In the measurement error correction device, the operation inspection section may determine whether or not the bio-measurer ordinarily operates based on whether or not the third current value is in a threshold current range. According to an embodiment, the third resistance value of the third simulator may be greater than the first resistance value and smaller than the second resistance value.

The measurement error correction device may further include a mode control section selecting the error correction mode and the user mode, wherein the correction section operates in the error correction mode and the operation inspection section operates in the user mode.

In the measurement error correction device, the mode control section may perform operation control in the error correction mode when electric power is initially applied to the bio-measurer after the bio-measurer is fabricated and performs operation control in the user mode when the bio-measurer is turned on after the measurement error of the bio-measurer is corrected in the error correction mode.

It is preferable that the operation inspection section may output an abnormality message to a user when the bio-measurer abnormally operates in the user mode.

**Advantageous Effects**

The measurement error correction device of a bio-measurer according to an embodiment of the present disclosure has a variety of effects as follows.

First, the measurement error correction device according to the present disclosure is disposed within the bio-measurer and serves to automatically correct a measurement error when electric power is initially applied to the bio-measurer after the fabrication of the bio-measurer. It is thereby possible to reduce time and cost spent for correcting the measurement error in the fabrication of the bio-measurer.

Second, the measurement error correction device according to the present disclosure inspects whether or not the bio-measurer ordinarily operates in an early stage of the operation of the bio-measurer, automatically or by a user selection, whereby the user can recognize whether or not the bio-measurer ordinarily operates.

Third, in the measurement error correction device according to the present disclosure, the resistance of the simulator used in correcting the measurement error of the bio-measurer and the resistance of the simulator used in inspecting whether or not the bio-measurer ordinarily operates are set different from each other. It is thereby possible to accurately correct the measurement error and accurately determine whether or not the bio-measurer ordinarily operates.

## DESCRIPTION OF DRAWINGS

FIG. 1 is a flowchart illustrating a related-art process of correcting a measurement error of a blood glucose meter;
FIG. 2 is a functional block diagram illustrating a measurement error correction device according to the present disclosure;
FIG. 3 illustrates an example of an abnormality message according to the present disclosure;
FIG. 4 is a flowchart illustrating the operation of the measurement error correction device in an error correction mode;
FIG. 5 illustrates an example of a correction slope and a correction size;
FIG. 6 is a flowchart illustrating the operation of the measurement error correction device in a user mode; and
FIG. 7 illustrates an example of a first resistance value R1, a second resistance value R2, and a third resistance value R3, as well as measurable current values corresponding to the resistance values.

## MODE FOR INVENTION

Hereinafter, reference will be made to a measurement error correction device of a bio-measurer according to the present disclosure in conjunction with the accompanying drawings.

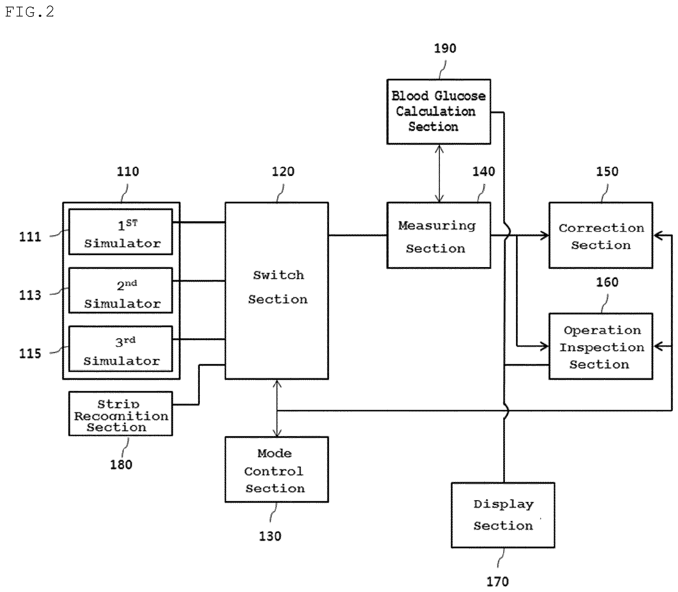

FIG. 2 is a functional block diagram illustrating a measurement error correction device according to the present disclosure.

Describing in more detail with reference to FIG. 2, a mode control section 130 controls a switch section 120 to select an error correction mode or a user mode. The error correction mode refers to a mode in which a measurement error of a bio-measurer is corrected at a point in time in which the bio-measurer is fabricated. In the user mode, a user inspects whether or not a bio-measurer ordinarily operates before measuring biometric values, such as a level of blood glucose, using the bio-measurer after purchasing the bio-measurer.

It is preferable that the mode control section 130 automatically controls the bio-measurer in the error correction mode when electric power is initially supplied to the bio-measurer after the fabrication of the bio-measurer is completed or controls the bio-measurer in the error correction mode when a user command for selecting the error correction mode is input.

The switch section 120 controls a simulator among a plurality of simulators to be selectively connected to a measuring section 140 depending on a mode selected by the mode control section 130. More specifically, when the error correction mode is selected by the mode control section 130, the switch section 120 sequentially connects a first simulator 111 and a second simulator 113 to the measuring section 140 under the control of the mode control section 130. When the user mode is selected by the mode control section 130, the switch section 120 connects a third simulator 115 to the measuring section 140 under the control of the mode control section 130. In addition, when a measurement mode for measuring the biometric values of a user using a sensor strip is selected, the mode control section 130 connects a strip recognition section 180 to the measuring section 140 under the control of the mode control section 130. Here, the strip recognition section 180 means a jig to which the sensor strip is fitted.

Describing the simulators 110 in more detail, the first simulator 111 has a first resistance value, the second simulator 113 has a second resistance value, and the third simulator 115 has a third resistance value. According to an embodiment of the simulators of the present disclosure, the third simulator 115 may not be provided, and one of the first simulator 111 and the second simulator 113 may be used as the third simulator 115. However, according to an exemplary embodiment of the simulators of the present disclosure, the third simulator 115 having a resistance value different from that of the first simulator 111 or the second simulator 113 is provided. In this case, the third resistance value is characterized by being greater than the first resistance value and smaller than the second resistance value.

In the error correction mode, the measuring section 140 applies electric power to the first simulator 111 and measures a first current value depending on the first resistance value of the first simulator 111 and the size of electric power applied to the first simulator. Sequentially, the measuring section 140 applies electric power to the second simulator 113 and measures a second current value depending on the second resistance value of the second simulator 113 and the size of electric power applied to the second simulator.

In the error correction mode, a correction section 150 corrects errors of the bio-measurer using the first current value and the second current value. The correction section 150 calculates correction coefficients indicating a correction slope and a correction size using the first resistance value, a theoretical first current value corresponding to the size of applied electric power, a measured first current value, the second resistance value, a theoretical second current value corresponding to the size of applied electric power, and a measured second current value.

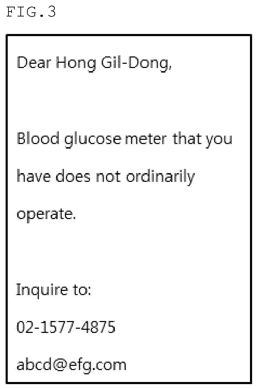

In the user mode, the measuring section 140 applies electric power to the third simulator 115 and measures a third current value depending on the third resistance value of the third simulator 115 and the size of electric power applied to the third simulator 115. An operation inspection section 160 determines whether or not the bio-measurer ordinarily operates using the third current value. When the third current value is in the threshold current range, the operation inspection section 160 determines that the bio-measurer ordinarily operates. When the third current value is out of the threshold current range, the operation inspection section 160 determines that the bio-measurer does not ordinarily operate and then outputs an abnormality message on a display section 170 to notify the user of the abnormality of the bio-measurer. FIG. 3 illustrates an example of the abnormality message according to the present disclosure. As illustrated in FIG. 3, a phrase indicating the abnormality of the bio-measurer, as well as contact information, is output. The contact information includes a phone number and an e-mail address, with which after services (A/S) for the bio-measurer are inquired.

In addition, in the measurement mode, a blood glucose calculation section 190 calculates the level of blood glucose of the user based on a current value measured using the measuring section 140. The blood glucose calculation section 190 corrects the measured current value using correction coefficients. The calculated level of blood glucose of the user is output on the display section 170.

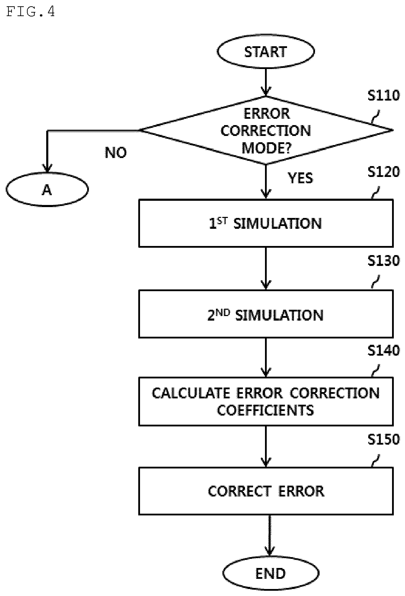

FIG. 4 is a flowchart illustrating the operation of the measurement error correction device in the error correction mode.

Describing in more detail with reference to FIG. 4, the measurement error correction device determines whether or not the mode is an error correction mode (S100). In the case of the error correction mode, the first simulator is operated to measure a first current value (S120). In sequence, the second simulator is operated to measure a second current value (S130).

As an example of determining the error correction mode, when electric power is initially applied to the bio-measurer, it may be determined to be the error correction mode. As another example of determining the error correction mode, when a user command for the error correction mode is input, the mode is determined to be the error correction mode.

Correction coefficients for a measurement error are calculated using the first current value and the second current value that have been measured (S140). The correction coefficients are calculated by substituting the measured first current value, a theoretical first current value, the measured second current value, and a theoretical second current value to correction equations in Formula 1.

[Formula 1]

$$Y_1 = aX_1 + b$$
$$Y_2 = aX_2 + b$$

In Formula 1, $X_1$ and $X_2$ are the first and current values that have been actually measured, $Y_1$ and $Y_2$ are theoretical first and second current values, a is a correction coefficient indicating a correction slope of the bio-measurer, and b is a correction coefficient indicating a correction size of the bio-measurer.

The correction error of the bio-measurer is corrected using the correction coefficients that have been calculated (S150).

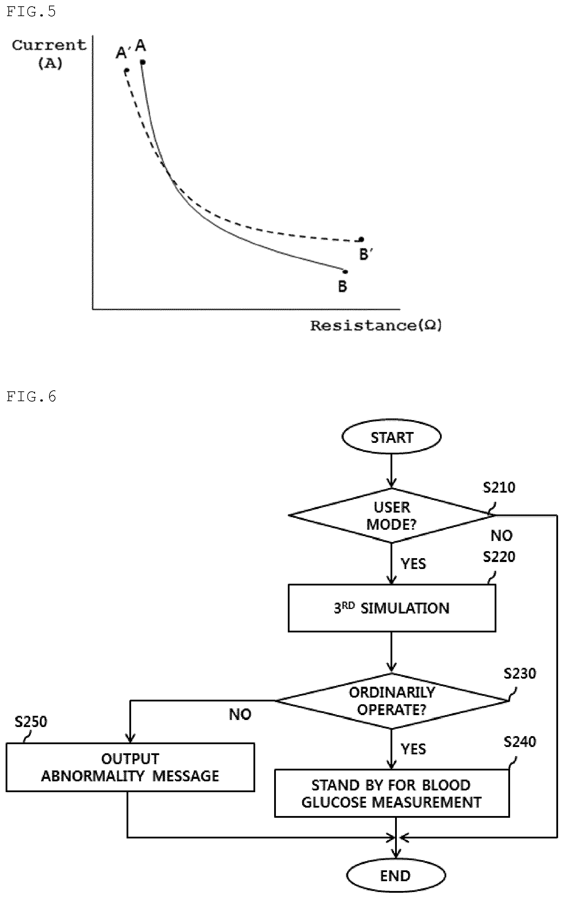

FIG. 5 illustrates an example of a correction slope and a correction size, in which a solid line indicates a theoretical curve produced from theoretical current values, and a dotted line indicates a measurement curve produced from measured current values. In FIG. 5, first, the x axis indicates resistance values, and the y axis indicates current values. First, a correction slope "a" is calculated such that the slope of the measurement curve is equal to the slope of the theoretical curve by calculating the slopes between end points A and B of the theoretical curve and end points A' and B' of the measurement curve. Afterwards, a correction size "b" is calculated by calculating a y-axis intercept value. In this manner, the measurement curve is corrected to be similar to the theoretical curve.

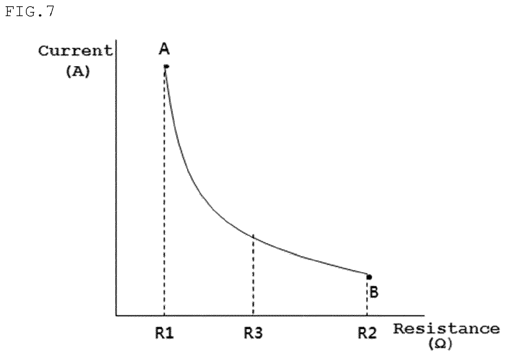

FIG. 7 illustrates an example of a first resistance value R1, a second resistance value R2, and a third resistance value R3, as well as measurable current values corresponding to the resistance values.

As illustrated in FIG. 7, the first resistance value is the lowest resistance that can be measured by the bio-measurer, and the third resistance value is the highest resistance that can be measured by the bio-measurer. A highest current value that can be measured corresponds to the first resistance value, while a lowest current value that can be measured corresponds to the third resistance value. Here, the higher the current value is, the higher the level of blood glucose is calculated. The lower the current value is, the lower the level of blood glucose is calculated. The third resistance value is between the first resistance value and the second resistance value, and is set as a resistance value with which a normal level of blood glucose of the user is to be measured. It is preferable that the third resistance value is set as a resistance value with which current values corresponding to blood glucose levels in the range of 50 mg/dL to 400 mg/dL with respect to applied electric power are output.

A measurement error is corrected by adjusting the end points of the measurement curve to be identical to the end points A and B of the theoretical curve using the current values based on the first resistance value and the second resistance value. In sequence, it is determined whether or not the bio-measurer having the corrected measurement error ordinarily operates based on whether or not the current value corresponding to the third resistance value is in the threshold current range. In this manner, the measurement error of the bio-measurer can be corrected more accurately. In addition, when a user applies electric power to a bio-measurer to measure the level of blood glucose of the user after purchasing the bio-measurer, the process of determining whether or not the bio-measurer ordinarily operates based on whether or not the current value corresponding to the third resistance value is in the threshold current range is performed automatically or in response to a user command. According to the present disclosure, the third simulator 115 can be used to correct a measurement error in the fabrication process and determine whether or not the bio-measurer ordinarily operates after the fabrication.

FIG. 6 is a flowchart illustrating the operation of the measurement error correction device in the user mode.

Describing in more detail with reference to FIG. 6, the measurement error correction device determines whether or not the mode is a user mode (S210). In the case of the user mode, a third current value is measured by operating the third simulator (S220). As an example of determining the user mode, when the mode is not determined to be the error correction mode after electric power is applied to the bio-measurer, it is unconditionally determined to be the user mode. As another example of determining the user mode, when a user command for the user mode is input, the mode is determined to be the user mode.

The measurement error correction device determines whether or not the bio-measurer ordinarily operates by determining whether or not the third current value is in the threshold current range (S230). When the bio-measurer is determined to ordinarily operate due to the third current value being in the threshold current range, the measurement error correction device comes into a measurement standby state and, when the sensor strip is fitted, calculates the biometric value of the user in the measurement mode (S240).

However, when the bio-measurer is not determined to ordinarily operate due to the third current value being out of the threshold current range, an abnormality message is output to the user (S250).

The above-described embodiments of the present disclosure can be recorded as programs executable by a computer, and can be realized in a general purpose computer that executes the program using a computer readable storage medium.

Examples of the computer readable storage medium include a magnetic storage medium (e.g. a floppy disk or a hard disk), an optical storage medium (e.g. a compact disc read only memory (CD-ROM) or a digital versatile disc (DVD)), and a carrier wave (e.g. transmission through the Internet).

While the present disclosure has been described with reference to the certain exemplary embodiments shown in the drawings, these embodiments are illustrative only. Rather, it will be understood by a person skilled in the art that various modifications and equivalent other embodiments may be made therefrom. Therefore, the true scope of the present disclosure shall be defined by the concept of the appended claims.

**Claims**

1. A measurement error correction device of a bio-measurer, the device comprising:

   a first simulator having a first resistance value;
   a second simulator having a second resistance value;
   a measurement section measuring a first current value by applying electric power to the first simulator and measuring a second current value by applying electric power to the second simulator; and
   a correction section correcting a measurement error of the bio-measurer using the first current value and the second current value.

2. The measurement error correction device according to claim 1, wherein the correction section corrects the measurement error of the bio-measurer by measuring the first current value and the second current value in an error correction mode in which an error of the bio-measurer is corrected.

3. The measurement error correction device according to claim 2, wherein the correction section calculates a correction slope and a correction size from the measured first current value, the measured second current value, a first theoretical current value corresponding to the first resistance value and applied electric power, and a second theoretical current value corresponding to the second resistance value and applied electric power.

4. The measurement error correction device according to claim 3, further comprising:

   a third simulator having a third resistance value; and
   an operation inspection section measuring a third current value by applying electric power to the third simulator and inspecting whether or not the bio-measurer ordinarily operates based on the third current value in a user mode in which it is inspected whether or not the bio-measurer ordinarily operate.

5. The measurement error correction device according to claim 4, wherein the first simulator, the second simulator, the measurement section, the correction section, and the third simulator of the measurement error correction device are disposed within the bio-measurer.

6. The measurement error correction device according to claim 5, wherein the operation inspection section determines whether or not the bio-measurer ordinarily operates based on whether or not the third current value is in a threshold current range.

7. The measurement error correction device according to claim 5, wherein the third resistance value of the third simulator is greater than the first resistance value and smaller than the second resistance value.

8. The measurement error correction device according to claim 5, further comprising a mode control section selecting the error correction mode and the user mode, wherein the correction section operates in the error correction mode and the operation inspection section operates in the user mode.

9. The measurement error correction device according to claim 8, wherein the mode control section performs operation control in the error correction mode when electric power is initially applied to the bio-measurer after the bio-measurer is fabricated and performs operation control in the user mode when the bio-measurer is turned on after the measurement error of the bio-measurer is corrected in the error correction mode.

10. The measurement error correction device according to claim 9, wherein the operation inspection section outputs an abnormality message to a user when the bio-measurer abnormally operates in the user mode.

FIG.1

```
            ┌─────────┐
            │  START  │
            └────┬────┘
                 │                    S10
                 ▼
    ┌──────────────────────────┐
    │        FABRICATE         │
    │   BLOOD GLUCOSE METER    │
    └────────────┬─────────────┘
                 │                    S20
                 ▼
    ┌──────────────────────────┐
    │  CORRECT MEASURED VALUE  │
    └────────────┬─────────────┘
                 │                    S30
                 ▼
    ┌──────────────────────────┐
    │  SELL BLOOD GLUCOSE METER │
    └────────────┬─────────────┘
                 │
                 ▼
            ┌─────────┐
            │   END   │
            └─────────┘
```

FIG.2

FIG.3

Dear Hong Gil-Dong,

Blood glucose meter that you

have does not ordinarily

operate.

Inquire to:

02-1577-4875

abcd@efg.com

FIG.4

```
                          ┌─────────┐
                          │  START  │
                          └─────────┘
                               │
                               ▼              S110
                        ╱──────────────╲
              ┌─────────    ERROR        
              │         CORRECTION        
        NO    │         ╲   MODE?    ╱
        ┌─────┘          ╲──────────╱
        ▼                     │ YES       S120
    ╭───────╮                 ▼
    │   A   │         ┌──────────────────┐
    ╰───────╯         │  1ST SIMULATION  │
                      └──────────────────┘
                               │           S130
                               ▼
                      ┌──────────────────┐
                      │  2ND SIMULATION  │
                      └──────────────────┘
                               │           S140
                               ▼
                      ┌──────────────────────┐
                      │ CALCULATE ERROR      │
                      │ CORRECTION           │
                      │ COEFFICIENTS         │
                      └──────────────────────┘
                               │           S150
                               ▼
                      ┌──────────────────┐
                      │  CORRECT ERROR   │
                      └──────────────────┘
                               │
                               ▼
                          ┌─────────┐
                          │   END   │
                          └─────────┘
```

11

FIG.5

FIG.6

FIG.7

## INTERNATIONAL SEARCH REPORT

| International application No. |
| --- |
| **PCT/KR2015/005297** |

**A. CLASSIFICATION OF SUBJECT MATTER**

*G01R 31/01(2006.01)i*

According to International Patent Classification (IPC) or to both national classification and IPC

**B. FIELDS SEARCHED**

Minimum documentation searched (classification system followed by classification symbols)
G01R 31/01; G01N 33/49; G01N 33/48; G01N 33/53; G01N 33/50; G01N 30/74

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: electric current, measurement, compensation, simulator

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2009-0033065 A (PHILOSYS CO., LTD.) 01 April 2009<br>See paragraphs [0042]-[0043]; figure 2; and claim 1 | 1-2 |
| A | | 3-10 |
| A | KR 10-2010-0006400 A (ALL MEDICUS CO., LTD.) 19 January 2010<br>See paragraphs [0026], [0031], [0038]-[0040]; figure 3; and claim 1 | 1-10 |
| A | KR 10-0903972 B1 (ALL MEDICUS CO., LTD.) 25 June 2009<br>See abstract; paragraphs [0001], [0035]-[0037], [0041]-[0043], [0068]-[0071]; figure 7;<br>and claim 1 | 1-10 |

☐ Further documents are listed in the continuation of Box C. ☒ See patent family annex.

| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |

| Date of the actual completion of the international search | Date of mailing of the international search report |
| --- | --- |
| 10 JULY 2015 (10.07.2015) | **13 JULY 2015 (13.07.2015)** |

| Name and mailing address of the ISA/**KR**<br>Korean Intellectual Property Office<br>Government Complex-Daejeon, 189 Seonsa-ro, Daejeon 302-701,<br>Republic of Korea<br>Facsimile No. 82-42-472-7140 | Authorized officer<br><br><br>Telephone No. |
| --- | --- |

Form PCT/ISA/210 (second sheet) (July 2009)

INTERNATIONAL SEARCH REPORT
Information on patent family members

International application No.

**PCT/KR2015/005297**

| Patent document cited in search report | Publication date | Patent family member | Publication date |
|---|---|---|---|
| KR 10-2009-0033065 A | 01/04/2009 | EP 2193367 A2<br>US 2010-0206749 A1<br>US 8343331 B2<br>WO 2009-041782 A2<br>WO 2009-041782 A3 | 09/06/2010<br>19/08/2010<br>01/01/2013<br>02/04/2009<br>14/05/2009 |
| KR 10-2010-0006400 A | 19/01/2010 | NONE | |
| KR 10-0903972 B1 | 25/06/2009 | NONE | |

Form PCT/ISA/210 (patent family annex) (July 2009)